# EUROPEAN PATENT APPLICATION

(11) **EP 0 523 292 A1**
(43) Date of publication of application: **20.01.1993**
(21) Application number: 91306427.5
(22) Date of filing: 16.07.1991
(51) Int. Cl.: A61G 12/00, A61F 4/00, A47G 21/08, B25J 19/02, B25J 9/16

(54) **Robotic feeding apparatus**

(71) Applicant: Topping, Michael John, Newcastle, Staffordshire ST5 6PD (GB)
(72) Inventor: Topping, Michael John, Newcastle, Staffordshire ST5 6PD (GB)
(74) Representative: Craske, Stephen Allan

(57) **Abstract**

A robotic arm 1 moves a cranked spoon 2 under control of a control unit 3 to scoop food from a dish 6 and transfer it to an eating position E adjacent to the mouth of a user. A display screen 4 has a series of columns representing adjacent strips of the dish, and the control unit 3 moves a cursor to each of the columns in turn. A switch 5 enables a user to select the column concurrently associated with the cursor and the control unit 3 then moves the arm 1 to remove a scoop of food from the corresponding area of the dish and transfer it to the eating position. The columns are further divided into segments and the arm removes food from each of the segments in turn, always starting at the same position in each column. Instead of a display screen a box carrying a series of sequentially illuminated lamps can be placed adjacent to the dish, and the switch can be operated to select the appropriate strip in similar manner.

## Description

### TECHNICAL FIELD OF THE INVENTION

This invention relates to eating apparatus which incorporates a robotic arm for transferring food from a container such as a bowl, plate or dish (which will hereinafter be referred to simply as a "dish") to the mouth of a person, who may be disabled.

### BACKGROUND

Such robotic eating aids have been proposed in the past, but in general these are fairly crude devices which scoop food from the dish with no provision for fine control. These devices are thus limited to use with liquid or semi-liquid food, and are, on the whole, not capable of being adapted to the individual requirements of a specific user.

A general aim of the present invention is to provide a form of robotic eating apparatus which enables a person, who may have a severe physical handicap, to have a high degree of control over the eating process.

### SUMMARY OF THE INVENTION

The present invention proposes eating apparatus comprising a robotic arm, control means for moving the arm to scoop food from a dish and transfer it to an eating position adjacent to the mouth of a user, a display device comprising a series of indicator elements representing adjacent areas of the dish, the control means being arranged to sequentially distinguish each of the indicator elements in turn, and a user-input device by which the user can command the control means to select whichever one of the indicator elements is concurrently distinguished, upon which the control means operates the arm to remove a scoop of food from the corresponding area of the dish and transfer it to the eating position.

The device can thus be used with relatively solid food, with different kinds of food being placed in different areas of the dish, and the user can select which kind of food he/she wishes to take next. The user-operable device will usually take the form of a single electrical switch, so that the apparatus can be used even by people with severely restricted movement and/or poor motor control.

The adjacent areas into which the dish is divided may conveniently be in the form of strips. Each strip is preferably sub-divided into segments, and the control means operates the arm according to the user's choice to remove food from the segments one after another. In order to ensure that the dish is cleared with little wastage it is desirable for the arm to take the first scoop from a segment at one end of the strip, the next scoop from that area from the next adjacent segment, and so on until the whole strip has been cleared. The spoon preferably starts in substantially the same position each time a scoop is taken from a particular strip.

In a simple embodiment the elements of the display device may simply be arranged to be placed immediately adjacent to the dish in use, and the display device comprises a row of indicator elements each associated with an adjacent area of the dish, the control means being arranged to sequentially operate the indicator elements. This may be placed immediately adjacent to the dish, and has the additional advantage of relatively low cost. In this embodiment the indicator elements may for example be in the form of filament lamps, LEDs or liquid crystal devices.

In a further embodiment the indicator elements of the display device may be comprised in a display screen, for example having a matrix of columns which are further sub-divided into segments corresponding to the segments of the dish. The segments are preferably highlighted before the food has been taken from the corresponding segment of the dish, but when the arm has taken the food from each segment the corresponding segment of the display screen ceases to be highlighted. The term "highlighted" is intended to cover a situation where the segment in question is illuminated brighter than the others, is illuminated where the others are not, or is otherwise made more visible than the other segments.

The columns may be sequentially distinguished by a cursor element which moves from column to column in a repeated sequence.

The display screen preferably includes an indicator element which, when selected by the user-input device, causes the screen to display a menu of control parameters for the robotic arm which are sequentially distinguished allowing the user to select one of the control parameters by the user-input device. As an alternative, the apparatus may include a programming keypad by which control parameters for the robotic arm can be reset.

The control parameters for the robotic arm preferably include the eating position and the speed at which the indicator elements are sequentially scanned.

The display device may include an indicator element which, when selected by the user-input device, allows the display to be reset when the dish is changed.

In the display screen version the indicator elements which allow resetting of the display device and resetting of the control parameters may be one and the same, i.e. the same column. When this element is selected the user may then be given a further choice of the two, or more, options, namely resetting the display or the control parameters.

The control unit preferably includes a microprocessor and a re-writable memory.

The arm preferably comprises a spoon of cranked shape.

### BRIEF DESCRIPTION OF THE DRAWINGS

The invention is exemplified in the accompanying drawings, in which:
Figure 1 is a schematic representation of robotic eating apparatus of the invention,
Figure 2 shows, in plan view, a dish for use with the apparatus of Fig. 1,
Figure 3 shows the display area of a display screen included in the apparatus of Fig. 1,
Figure 4 is a schematic representation of another form of robotic eating apparatus of the invention,
Figure 5 is an electrical block diagram of the apparatus of Fig. 4, and
Figure 6 shows the display device of the apparatus of Fig. 4, together with a plan view of a dish for use with the apparatus.

### DETAILED DESCRIPTION OF THE DRAWINGS

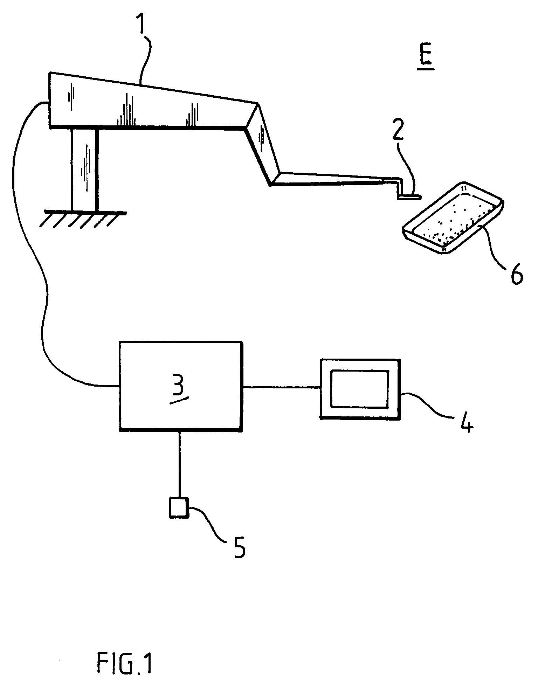

Referring firstly to Fig. 1, the apparatus comprises a robotic arm 1 which carries a cranked spoon 2 and is capable of moving the spoon in three mutually perpendicular axes. Movements of the arm are controlled by a central control unit 3 which incorporates a microprocessor working with custom-made software. The control unit is linked to a display screen 4, which may be of the cathode ray tube type but is preferably of the liquid crystal display type.

Command signals are input to the control unit by a user-operable switch 5.

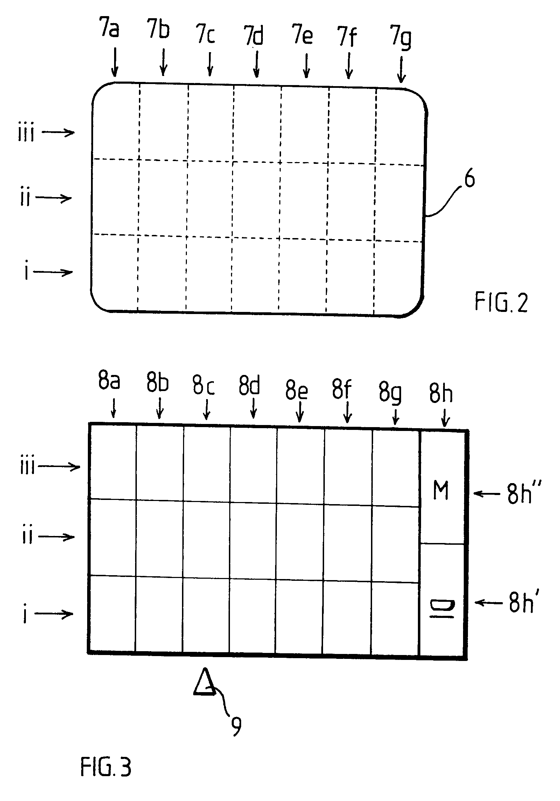

The control unit 3 can move the arm to scoop food from a dish 6 and transfer it to an eating position E adjacent to the mouth of a user. Normally, the dish will be of shallow, generally rectangular shape, as shown in Fig.s 1 and 2. The top area of the dish is notionally divided into a series of adjacent strips 7a-g, which in the present example are seven in number. Each strip extends parallel with the shorter edges of the dish, and is placed so that when the spoon is in the vicinity of the dish the strips 7a-g are all substantially aligned with the axis of the arm 1. Each strip is in turn notionally sub-divided into three (and possibly more) segments, which in Fig. 2 are designated by the suffixes i, ii and iii from front to rear of the dish.

Referring now to Fig.3, the display screen 4 displays a matrix of eight columns 8a-h. The first seven columns 8a-g (from left to right) represent the strips 7a-g of the dish, and each column is in turn sub-divided vertically into three segments i-iii from bottom to top. The eighth column 8h is sub-divided into two upper and lower segments 8h'' and 8h' respectively, the function of which will be explained below. Below each column there is displayed an arrow-shaped cursor 9 which can move to a position below any of the eight columns.

At the start of an operating sequence all 23 segments of the matrix exhibited on the display screen are visible, and the cursor 9 sequentially traverses from column to column in a left-to-right direction, although it could equally well move in the opposite direction. When it reaches the eighth column 8h the cursor 9 returns to the first column 8a to start the sequence over again. Whilst the cursor is in position under any of the eight columns the user can operate the switch 5 to tell the control means that the user has selected the column which the cursor is below at that particular instant. For the purpose of example let us suppose that column 8c has been selected. The control means then operates the arm 1 to move the spoon 2 into position adjacent to the dish 6 and then lower the spoon onto the surface of the dish at the front end of the corresponding strip 7c. The arm then moves the spoon along segment 7ci of the respective strip to take a scoop of food from that segment. At the end of that segment the arm lifts the spoon together with the scoop of food, and transfers it to the eating position E so that the user can take the food into his/her mouth.

By this time the cursor 9 has recommenced its sequential scanning of the columns 8a-h, but the segment 8ci corresponding to the segment 7ci from which the food has been removed has now been extinguished, i.e. is blank, to indicate that food is no longer available at this position. The user can now again select any of the columns using the switch 5. If he/she now selects a different column the arm will remove a scoop of food from the corresponding front segment i of the dish in the manner described, and the selected segment will again be extinguished on the screen 4. However, if the user selects the same column 8c the arm will lower the spoon into the same position on the dish at the front end of strip 7c, but on this occasion the spoon will be moved further forward to take a scoop from the middle segment 7cii. By starting from the same position each time a particular column is selected it is ensured that most of the food can be cleared from a column with little wastage. The device can be used with relatively solid food, with different kinds of food being placed in different areas of the dish.

The process of selecting columns can be continued until the entire dish is cleared. When the third segment of each column has been selected that entire column will become blank, and the cursor will then skip that column in subsequent scanning sequences.

When all the food has been removed from the dish, or if the user has had sufficient, the dish can be removed and replaced with a new dish containing the next course. By operating the switch 5 at the appropriate time the user can select the eighth column 8h. The two segments 8h' and 8h'' are then alternately illuminated, and the user may then select segment 8' by operating switch 5 whilst that segment is illuminated. This commands the control unit to reset the matrix with all 23 segments illuminated so that the user can now commence taking food from the new dish in the manner described.

If, at any time, the user opts to select segment 8'' the screen displays a menu of control parameters for the arm 1. These again have a sequentially scanning cursor enabling the user to select options of his choice using the switch 5. By making selections from appropriate sub-menus the user can control the speed at which the cursor scans the columns, and adjust the parameters of the arm to vary the eating position for example. Upon leaving this menu the user is given a choice of storing the newly entered parameters as the default parameters which the apparatus adopts each time it is switched on, or simply using them as temporary settings.

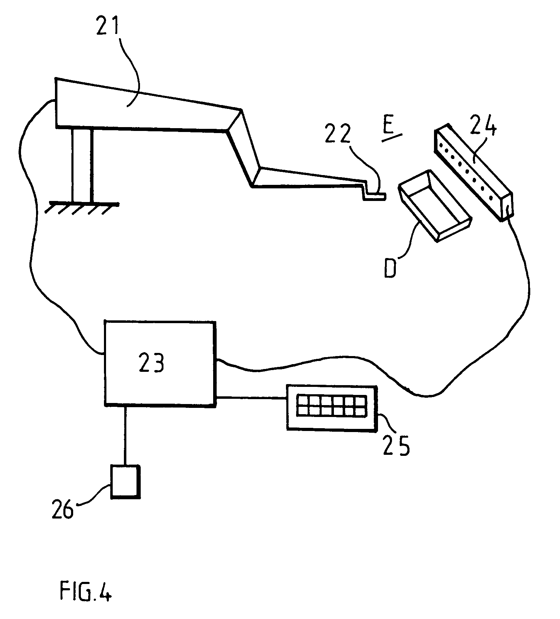

The apparatus of Fig. 4 again comprises a robotic arm 21 which carries a cranked spoon 22 and is capable of moving the spoon in three mutually perpendicular axes. Movements of the arm are controlled by a central control unit 23, such that the control unit can move the arm to scoop food from a dish D and transfer it to an eating position E adjacent to the mouth of a user. The control unit is connected to a display box 24 and a programming keypad 25. Command signals are sent to the control unit from a user-input switch 26.

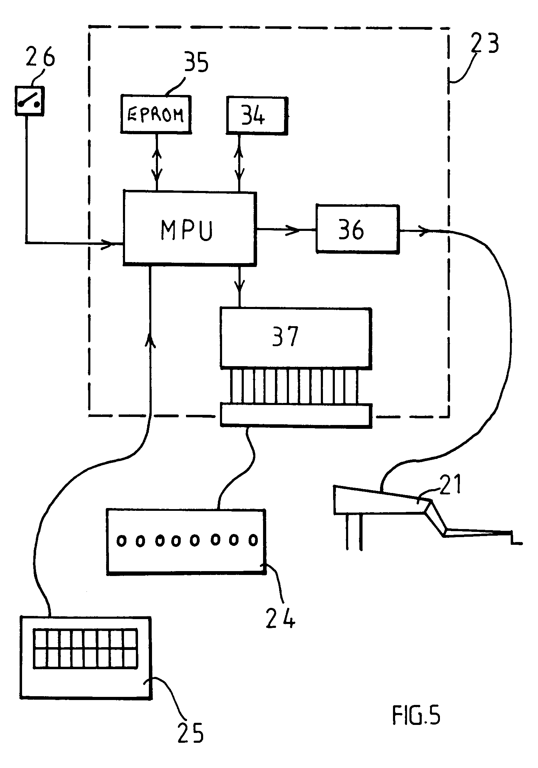

The control unit is shown in more detail in Fig. 5 and has at its heart a microprocessor MPU which operates using custom-made software contained in a PROM or similar non-erasable memory 34. The microprocessor receives signals from the user-input switch 26 and the programming keys 25 (see below), and a re-writable non-volatile memory in the form of an EPROM 35 is connected to the microprocessor to store various re-writable parameters The microprocessor controls the arm 21 via an interface unit 36 and drives the display box 24 via a decoder and driver unit 37. The arm 21, switch 26, keypad 25 and control box 24 are all connected to the control unit 23 via releasable plug and socket connectors, and the arm 21 also receives power from the control unit via the same connector.

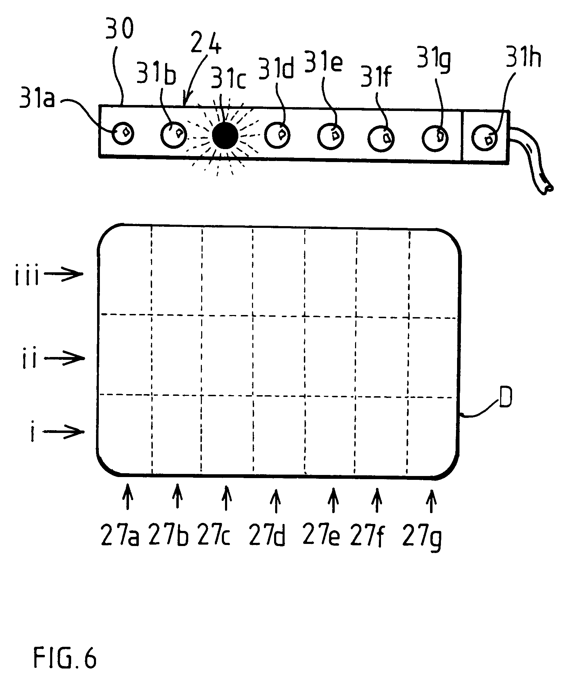

Generally, the dish D will be of shallow, generally rectangular shape, as shown in Fig. 6. The top area of the dish is notionally divided into a series of adjacent strips 27a-g, which in the present example are seven in number. Each strip extends parallel with the shorter edges of the dish, and is placed so that when the spoon is in the vicinity of the dish the strips 27a-g are all substantially aligned with the axis of the arm 21. Each strip is in turn notionally sub-divided into three (and possibly more) segments, which are designated by the suffixes i, ii and iii from front to rear of the dish.

The display device 24, which is also shown in detail in Fig. 6, includes a housing 30 having a bank of eight light-emitting diodes (LEDs) 31a-h. The housing can be placed adjacent to one of the longer edges of the dish D with one of the first seven LEDs 31a-g at the head of each strip 27a-g. The LEDs are sequentially illuminated by the control unit 23, and whilst any one of the LEDs is illuminated the user can operate the switch 26 to tell the control unit 23 that the user has selected the corresponding strip 27a-g. For the purpose of example let us suppose that LED 31c has been selected. The control means then operates the arm 21 to move the spoon 22 into position adjacent to the dish D and then lower the spoon onto the surface of the dish at the front end of the corresponding strip 27c. The arm then moves the spoon along segment 27ci of the respective strip to take a scoop of food from that segment. At the end of that segment the arm lifts the spoon together with the scoop of food, and transfers it to the eating position E so that the user can take the food into his/her mouth.

By this time the LEDs 31 have recommenced sequential illumination. The user can now again select any of the columns using the switch 26. If he/she now selects a different LED the arm will remove a scoop of food from the corresponding front segment i of the dish in the manner described. However, if the user selects the same LED 31c the arm will lower the spoon into the same position on the dish at the front end of strip 27c, but on this occasion the spoon will be moved further forward to take a scoop from the middle segment 27cii.

The process of selecting LEDs can be continued until the entire dish is cleared. When food has been removed from the third segment of each strip the corresponding LED will be skipped in subsequent scanning sequences.

When all the food has been removed from the dish, or if the user has had sufficient, the dish can be removed and replaced with a new dish containing the next course. By operating the switch 26 at the appropriate time the user can select the eighth lamp 31h. This allows the user to reset the apparatus for a fresh dish of food.

The programming keypad 25 has a number of keys which enable various control parameters to be reset, e.g. by a supervisor. Operation of a LEARN key sends a command to the microprocessor telling it that a new set of parameters are about to be input. The arm 21 can then be re-adjusted to a new mouth position E using additional keys such as BASE LEFT, BASE RIGHT, SHOULDER UP, SHOULDER DOWN, ELBOW IN, and ELBOW OUT. In addition, the speed at which the LEDs are scanned can be adjusted by pressing a SCAN SPEED key. In this mode the seven LEDs 31a-g represent a scanning speed scale with the fastest speed represented on the right and the slowest speed represented on the left. The LEDs then commence scanning, and by operating the switch 26 when the appropriate LED is lit the new scanning speed can be selected. Once the required mouth position and scanning speed have been achieved a SET key is pressed to write the newly set parameters into the EPROM 35, which becomes the default setting for future use.

The apparatus of Fig.s 4 to 6 is relatively inexpensive to manufacture compared with that of Fig.s 1 to 3.

In each case, the ability to control all the major functions using a simple switch enables the apparatus to be used even by people with severely restricted movement and/or poor motor control.

## Claims

1. Eating apparatus comprising a robotic arm (1), control means (3) for moving the arm to scoop food from a dish (6) and transfer it to an eating position adjacent to the mouth of a user, a display device (4) comprising a series of indicator elements representing adjacent areas of the dish, the control means being arranged to sequentially distinguish each of the indicator elements in turn, and a user-input device (5) by which the user can command the control means to select whichever one of the indicator elements is concurrently distinguished, upon which the control means operates the arm to remove a scoop of food from the corresponding area of the dish and transfer it to the eating position.

2. Eating apparatus according to Claim 1, in which the adjacent areas into which the dish is divided are in the form of strips.

3. Eating apparatus according to Claim 2, in which each strip is sub-divided into segments, and the control means operates the arm to remove food from the segments one after another.

4. Eating apparatus according to Claim 3, in which the control means operates the arm to take the first scoop from a segment at one end of the strip, the next scoop from that area from the next adjacent segment, and so on until the whole strip has been cleared.

5. Eating apparatus according to Claim 4, in which the control means operates the arm to start in substantially the same position each time a scoop is taken from a particular strip.

6. Eating apparatus according to any preceding claim, in which the display device is arranged to be placed immediately adjacent to the dish in use, and the display device comprises a row of indicator elements each associated with an adjacent area of the dish, the control means being arranged to sequentially operate the indicator elements.

7. Eating apparatus according to any of Claims 1 to 5, in which the indicator elements of the display device are comprised in a display screen,

8. Eating apparatus according to Claim 7, in which the indicator elements comprise a series of columns which are further sub-divided into segments corresponding to the segments of the dish.

9. Eating apparatus according to Claim 8, in which the segments are highlighted before the food has been taken from the corresponding segment of the dish, but when the arm has taken the food from each segment the corresponding segment of the display screen ceases to be highlighted.

10. Eating apparatus according to any of Claims 7 to 9, in which the display screen includes an indicator element which, when selected by the user-input device, causes the screen to display a menu of control parameters for the robotic arm which are sequentially distinguished allowing the user to select one of the control parameters by the user-input device.
